# EUROPEAN PATENT APPLICATION

(11) **EP 1 724 309 A1**
(43) Date of publication of application: **22.11.2006**
(21) Application number: 05101712.7
(22) Date of filing: 04.03.2005
(51) Int. Cl.: C09D 5/00, C11D 1/00, A61K 8/37, A61K 8/894, A61K 8/897, A61Q 17/00

(54) **Composition comprising a polyoxyethylene fluoro or siloxane surfactant**

(71) Applicant: Boon, Cornelis, 1531 MA WORMER (NL)
(72) Inventor: Boon, Cornelis, 1531 MA WORMER (NL)
(74) Representative: Beetz, Tom

(57) **Abstract**

The invention relates to a composition comprising:
a) an esterified fatty ester having 8 tot 18 C atoms;
b) a polyoxyethylene fluoro or siloxane surfactant;
c) an organic solvent for the esterified fatty ester; and
d) water.

The composition may comprise a further surfactant. The composition may be used to protect surfaces against dirt. More particularly the invention relates to a composition to protect pigeon feet against dirt, such as pigeon-dung.

## Description

The invention relates to a composition comprising a polyoxyethylene fluoro or siloxane surfactant, to the use of said composition in protecting surfaces, and to a method for treating surfaces.

The invention particularly relates to protection of pigeon feet against dirt and excrements.

Prior to a flight race for racing pigeons wherein the pigeons are transported or intended for transport from its pigeon house to be released so that it may freely fly back to its pigeon house or to any other destination, the pigeons are fed with extra amounts of minerals, cereals, vitamins, and the like, which increase the weight of the pigeons substantially. Thereafter the pigeons are put into a cage. Such cages can contain 25 to 40 pigeons, which mean that the pigeons have only limited room and cannot move easily. This situation can last for a few hours to several days. Due to this situation the pigeon feet are messed by excrements (pigeon-dung) of their own and of their fellow pigeons. These excrements stick to the feet and the weight thereof can amount up to several grams. These excrements have a detrimental effect on the speed performance of the racing pigeon in view of diminished aerodynamic properties and increased weight.

Pigeon keepers try to alleviate the sticking of pigeon-dung and other dirt by covering the pigeon feet with petroleum jelly. However, this method is deficient in that it is hardly possible to apply such jelly evenly over the feet, whereas petroleum jelly has the disadvantage of being sticky, thereby attracting other dirt, feathers, and the like.

About 15 years ago an improvement to petroleum jelly was proposed, being a composition comprising an esterified fatty ester having 8 tot 18 C atoms, an organic solvent for the esterified fatty ester, water, and a surfactant. This composition was brought onto the market under the trade name PS 90™. Some problems were solved by PS 90, but new problems occurred. PS 90 was applied onto the pigeon feet as a spray, but appeared not to be a commercial success due to limited attachment to the feet, limited working time of repellant activity, poor water repellant activity and poor moistening of the feet. There is thus a need for an improved composition that is devoid of these disadvantages.

An improved composition has now been found which is devoid of the above-mentioned disadvantages. To this end the invention pertains to a composition comprising:
a) an esterified fatty ester having 8 tot 18 C atoms;
b) a polyoxyethylene fluoro or siloxane surfactant;
c) an organic solvent for the esterified fatty ester; and
d) water.

It was found that said composition, which contains a polyoxyethylene fluoro or siloxane surfactant instead of, or in addition to, a conventional surfactant could easily be applied to pigeon feet in an even manner with superior water, dirt, and excretion repellant activity.

In a preferred embodiment the polyoxyethylene fluoro or siloxane surfactant is added in addition to the further (conventional) surfactant.

In another preferred embodiment the composition comprises 25-65 wt.%, preferably 40-50 wt.% esterified fatty ester; 0.01-15 wt.%, preferably 0.05-10 wt.% polyoxyethylene fluoro or siloxane surfactant; 10-30 wt.%, preferably 15-30 wt.% organic solvent; 5-45 wt.%, preferably 15-30 wt.% water; 0-25 wt.%, preferably 2-15 wt.% further surfactant; whereby the total of ingredients sums up to 100 wt.%.

A particularly preferred composition comprises 43 - 47 wt.% ethyl esterified fatty ester; 0.1 - 5. wt.% polyoxyethylene fluoro surfactant; 23 - 27 wt. % diethylether; 22-29 wt.% water; and the remainder being a further surfactant.

Preferably, the fatty acid is esterified with a C1-C6 alkyl group, more preferably methyl or ethyl group. The fatty acid may be any natural occurring saturated, unsaturated, branched or unbranched fatty acid. Natural and synthetic unsaturated fatty acids, as well as mixtures of various natural fatty acids, mixtures of various synthetic fatty acids, and mixtures of natural and synthetic fatty acids can be used. One type of unsaturated fatty acids useful herein is obtainable from natural drying oils. Typical, but non-limiting examples of fatty acids esters useful in the invention are fatty acids esters derived from fatty acids such as dehydrated castor oil fatty acid, linoleic acid, and linolenic acid. Further non-limiting examples of useful natural oil fatty acid esters are derived from tall oil fatty acid, sunflower oil fatty acid, corn oil fatty acid, cottonseed oil fatty acid, lard fatty acid, mustard seed fatty acid, olive oil fatty acid, palm oil fatty acid, peanut oil fatty acid, linseed oil fatty acid, soybean oil fatty acid, rapeseed oil fatty acid, rice bran oil fatty acid, safflower oil fatty acid, sesame oil fatty acid, and tallow fatty acid. Mixtures of fatty acid esters may also be used.

The organic solvent may be any suitable solvent, such as alcohols, ethers, ketones, aromatic solvents, halogenated solvents, and the like, and mixtures thereof. Diethyl ether is a particularly useful solvent for the fatty acids of the composition.

The further surfactant may be any conventional anionic, cationic, amphoteric, or nonionic surfactant. Surfactants that can be used are linear or branched alkali metal mono- and/or di-(C8-14) alkyl diphenyl oxide mono- and/or disulfonates, which are commercially available for example as DOWFAX® 3B-2 and DOWFAX® 2A-1. Other suitable surfactants include the primary alkyl sulfates, alkyl sulfonates, alkylaryl sulfates, sec-alkyl sulfates, alkyl phosphonates. Examples include sodium (C10-18) alkyl sulfates such as sodium dodecyl sulfate; sodium (C10-18) alkyl sulfonates such as sodium hexadecyl-1-sulfonate and sodium (C12-18) alkylbenzene sulfonates, such as sodium dodecylbenzene sulfonates. The corresponding potassium salts may also be employed.

Nonionic surfactants are well known in the art, for instance from ethyleneoxide to a fatty oxyalcohol, particularly with 12 to 18 carbon atoms, More particularly oxyalcohol-ethoxylate containing 2 to 50, preferably 6 to 8 mole of ethyleneoxide per mole of alcohol. Other nonionic surfactants are (C1-C20) alkylphenols or polypropoxylated or polyglycerolated fatty acids, having a fatty chain comprising, for example, from 8 to 18 carbon atoms, and it being possible for the number of glycerol groups to range in particular from 2 to 30. There may also be mentioned copolymers of ethylene and propylene oxide, condensates of ethylene and propylene oxide with fatty alcohols; polyethoxylated fatty amides preferably having from 2 to 30 mol of ethylene oxide, polyglycerolated fatty amides comprising on average 1 to 5 glycerol groups and in particular 1.5 to 4; polyethoxylated fatty amines preferably having 2 to 30 mol of ethylene oxide; ethoxylated fatty acid esters of sorbitan having from 2 to 30 mol of ethylene oxide; fatty acid esters of sucrose, fatty acid esters of polyethylene glycol, (C6-C24) alkyl polyglycosides, derivatives of N-(C6-C24) alkyl glucamine, amine oxides such as (C10-C14)alkylamine oxides or N-(C10-C14) acylaminopropylmorpholine oxides; and mixtures thereof.

The improved properties of the novel composition reside in the use of a polyoxyethylene fluoro or siloxane surfactant, which may be anionic, cationic, amphoteric, or nonionic. In most common nonionics, the polar group is the polyoxyethylene chain or polyol moiety. The isolated oxyethylene group -CH₂CH₂O- shows a dual hydrophilic-hydrophobic character. The hydrophobic part of nonionics can be a C6-22 hydrocarbon substituent: straight, branched, alkylaryl, saturated, or unsaturated; it can contain one or multiple hydrocarbon chains. Moreover, the hydrophobic part can be a fluoro- or perfluorocarbon-chain, polymeric siloxane with a wide range of structures, or a polyoxypropylene-polyoxybutylene chain. Suitable fluoro surfactants are commercially available at Chemguard, Inc, and at Sigma-Aldrich under the trade name ZONYL®.

The composition of the invention is harmless to humans and animals, and odorless soluble or emulsifyable in water. The material can be applied to body parts of humans or animals, and more generally to any surface, such as shoes, boots, garment, and the like. When applied to body parts, feet and legs are preferred because of their sensitivity to contamination with dirt, dung, humus, and the like. The composition can also be applied to skin, hands, and feet of humans. A particularly useful application is the use of the composition for incontinent patients. By applying the skin that comes in contact with excrements with the composition, such patients can be cleaned easily and undergo less irritation from aggressive components in excrement, which may lead to eczema and the like.

The composition can be applied by spraying, atomizing, dipping, rolling, brushing, and the like onto the surface to be treated.

## Claims

1. A composition comprising:
a) an esterified fatty ester having 8 tot 18 C atoms;
b) a polyoxyethylene fluoro or siloxane surfactant;
c) an organic solvent for the esterified fatty ester; and
d) water.

2. The composition of claim 1 further comprising a further surfactant.

3. The composition of claim 1 or 2, wherein the fatty acid is esterified with a C1-C6 alkyl group.

4. The composition of any one of claims 1-3, wherein the organic solvent is diethyl ether.

5. The composition of any one of claims 1-4,
a) comprising 25-65 wt.%, preferably 40-50 wt.% esterified fatty ester;
b) comprising 0.01-15 wt.%, preferably 0.05-10 wt.% polyoxyethylene fluoro or siloxane surfactant;
c) comprising 10-30 wt.%, preferably 15-30 wt.% organic solvent;
d) comprising 5-45 wt.%, preferably 15-30 wt.% water;
e) comprising 0-25 wt.%, preferably 2-15 wt.% further surfactant; and
wherein the total of ingredients sums up to 100 wt.%.

6. The composition of claim 6 comprising:
43 - 47 wt.% ethyl esterified fatty ester;
0.1 - 5. wt.% polyoxyethylene fluoro surfactant;
23 - 27 wt. % diethylether;
22-29 wt.% water; and
the remainder being a further surfactant.

7. Use of the composition of any one claims 1-6 for protecting a surface against dirt.

8. The use according to claim 7 wherein the surface is a pigeon foot.

9. Method for treating surfaces, in particular pigeon feet, by applying the composition of any one of claims 1-6 onto said surface, by spraying, atomizing, dipping, rolling, or brushing.
